# EUROPÄISCHE PATENTSCHRIFT

(11) **EP 2 547 644 B1**
(45) Veröffentlichungstag und Bekanntmachung des Hinweises auf die Patenterteilung: **30.07.2014**
(21) Anmeldenummer: 11707852.7
(22) Anmeldetag: 10.03.2011
(51) Int. Cl.: C07C 51/41, C07C 53/06

(54) **VERFAHREN ZUR STOFFLICHEN VERWERTUNG VON WÄSSRIGEN GEMISCHEN, DIE AMEISENSÄURE, FORMALDEHYD UND METHANOL ENTHALTEN**
METHOD FOR RECYCLING THE MATERIALS OF AQUEOUS MIXTURES COMPRISING FORMIC ACID, FORMALDEHYDE AND METHANOL
PROCÉDÉ DE RECYCLAGE DE SUBSTANCES DE MÉLANGES AQUEUX, CONTENANT DE L'ACIDE FORMIQUE, DU FORMALDÉHYDE ET DU MÉTHANOL

(30) Priorität: 17.03.2010 DE 102010011691
(43) Veröffentlichungstag der Anmeldung: 23.01.2013
(73) Patentinhaber: Addcon Europe Gmbh, 06749 Bitterfeld-Wolfen (DE)
(72) Erfinder: EDERLE-LERCH, Klaus, 83229 Aschau im Chiemgau (DE); KOCHANNEK, Bernd, 53113 Bonn (DE); STÜWE, Hans-Jürgen, 06905 Bad Schmiedeberg (DE)
(74) Vertreter: Von Kreisler Selting Werner - Partnerschaft von Patentanwälten und Rechtsanwälten mbB
(86) Internationale Anmeldenummer: PCT/EP2011/053642
(87) Internationale Veröffentlichungsnummer: WO 2011/113748

(56) Entgegenhaltungen:
- WO-A1-96/01248
- DE-A1- 4 126 730
- DE-C- 424 017
- US-A1- 2007 241 053
- N. A. ARISTOVA: THEORETICAL FOUNDATIONS OF CHEMICAL ENGINEERING, Bd. 37, Nr. 2, 1. Januar 2003 (2003-01-01) , Seiten 179-183, XP55009330, ISSN: 0040-5795, DOI: 10.1023/A:1023338907810

## Beschreibung

Gegenstand der Erfindung ist ein Verfahren zur Aufbereitung von Gemischen, die Ameisensäure, Formaldehyd und Methanol enthalten.

In vielfältigen industriellen Prozessen entstehen Nebenproduktströme, Destillate oder Filtrate, die Formaldehyd, Ameisensäure und Methanol in solchen Konzentrationen enthalten, die eine stoffliche Verwertung als Alternative zur Entsorgung wirtschaftlich sinnvoll erscheinen lassen. Diese Mischungen lassen sich nur mit hohem technischen Aufwand destillativ trennen, sich bildende Azeotrope erfordern sogar die Anwendung von Druckdestillationsverfahren mit entsprechend hohem Energieverbrauch. Durch die korrosiven Eigenschaften der Ameisensäure ist ein außerordentlich hoher Instandhaltungsaufwand notwendig und die Apparate müssen in hochwertigen und somit teuren Werkstoffen ausgeführt sein. Die Destillate enthalten in der Regel immer noch nennenswerte Anteile an Methanol, Formaldehyd und Ameisensäure.

Bei Formaldehydlösungen (15 bis 25 Gew.-%) mit vergleichsweise niedrigem Ameisensäure-(< 1 Gew.-%) und Methanolgehalt (< 5 %) können Ameisensäureanteile mittels Ionenaustauscher abgetrennt werden. Die vergleichsweise niedrigprozentigen Lösungen an Formiaten, die beim Regenerieren der Ionenaustauscher entstehen, sind in der Regel nicht wirtschaftlich verwertbar und müssen über die biologische Abwasserreinigungsanlagen mit entsprechend hohen CSB / BSB Lasten entsorgt werden. Die nach der Abtrennung der Ameisensäure resultierenden wässrigen Formaldehyd- / Methanolgemische können dann entweder destillativ gereinigt werden oder werden im Absorptionsteil von Formaldehydanlagen in dieser Konzentration wieder verwendet. Um bei der Destillation auf brauchbare Konzentrationsbereiche (> 50 Gew.-%) zu kommen, ist in der Regel eine Druckdestillation notwendig, die sowohl hohe spezifische Energieverbräuche hat, zu einem stark belasteten Destillatstrom (ca. 5 Gew.% Formaldehyd) führt und in welchem über oxydative Prozesse wiederum Ameisensäure gebildet werden. Bei der Wiederverwendung in der Formaldehyderzeugung ist typischerweise die Wasserbilanz des Gesamtverfahrens limitierend für die verwertbaren Mengen.

N. A. Aristova (Theoretical Foundations of Chemical Engineering, Bd. 37, Nr. 2, 1. Januar 2003, Seiten 179-183) und US 2007/241053 offenbaren Verfahren zur Aufarbeitung von Ameisensäure, Formaldehyd und Methanol enthaltenden Gemische. In Aristova wird das Gemisch einer oxidativen Behandlung umgesetzt. In US 2007/241053 wird das Gemisch in einem Bioreaktor mit Mikroorgansimen versetzt.

Alternativ kann der in der Lösung enthaltene Formaldehyd nach Entsäuerung mit Ammoniak in Hexamethylentetramin überführt werden, was aber Anbetracht der Mengenverhältnisse zu einem massiven Überangebot an Hexamethylentetramin führt. Wird die Umsetzung mit Ammoniak in Gegenwart von höheren Konzentrationen an Ameisensäure durchgeführt, dann bildet sich Ammoniumformiat, welches bei der Aufarbeitung im Rahmen der sog. Leuckhart-Wallach Reaktion (vgl. dazu Leuckart, R. Chemische Berichte 1885, 18, 2341) zu starker Nebenproduktbildung (aliphatische Amine) führt.

Arpeisensäurefösungen (Ameisensäureanteil 5 bis 75 Gew.%) mit vergleichsweise niedrigem Formaldehyd- und Methanolanteil (jeweils < 5 Gew.-%) können entweder destillativ mit sehr großem Aufwand und Energieverbrauch aufgearbeitet werden oder werden thermisch verwertet.

Die gezielte Herstellung von Kaliumformiat aus Formaldehydlösung mit niedrigen Ameisensäuregehalten ist in der Literatur beschrieben. WO 96/01248 beschreibt ein Verfahren zur Herstellung von Kaliumformiat aus wässriger Formaldehydlösung. Dieses Verfahren zeigt aber erhebliche Schwächen. Signifikant sind diese im Bereich der Reaktionssicherheit, da die wässrige Formaldehydlösung vorgelegt und Kaliumhydroxidlösung zudosiert wird. In Laborexperimenten führt diese Verfahrensweise zum erheblichen Risiko einer unkontrollierten (exothermen) Aldolreaktion. Das Fehlen eines Aldolisierungsinhibitors wirkt stich gleichfalls negativ auf die Qualität (Farbe, Geruch) der erzeugten Kaliumformiatlösung aus. Ferner spiegelt das beschriebene Verfahren eine Reaktionsführung wieder, die zu Zeiten entwickelt wurde, als Energieeffizienz noch nicht in diesem Maß die Verfahrensentwicklung beeinflusst hat. Zunächst wird nach dem Verfahren gemäß WO 96/01248 während der Zugabe der Lauge gekühlt - nicht zuletzt um auch die Gefahren einer Aldolisierung zurück zu drängen - und dann zur destillativen Entfernung von gebildetem Methanol wieder aufgeheizt.

In der DE 41 26 730 A1 wird ein Verfahren zur Herstellung von Calciumformiat aus verdünnter Formaldehydlösung beschrieben. Die in diesem Verfahren verwendeten Inhibitoren sind allerdings in recht aufwändigen Prozessen abzutrennen, die Reaktionsführung ist ebenfalls hinsichtlich der Energieverbräuche ineffizient.

Aufgabe der vorliegenden Erfindung ist die Bereitstellung eines kosten- und insbesondere energieeffizienten Verfahrens zur Aufbereitung von Gemischen, insbesondere wässrigen Gemischen, die Ameisensäure, Formaldehyd und Methanol enthalten.

Die Erfindung betrifft ein Verfahren zur Aufarbeitung von Gemischen, aus Ameisensäure, Formaldehyd, Methanol und gegebenenfalls Wasser enthalten.

Gegenstand der Erfindung ist in einer ersten Ausführungsform ein Verfahren zur Aufarbeitung von Gemischen, die Ameisensäure, Formaldehyd und Methanol enthalten, das dadurch gekennzeichnet ist, dass man den Ameisensäureanteil mit Lauge direkt in die entsprechenden Formiate und zeitgleich den Formaldehydanteil nach Disproportionierung in Gegenwart von Alkali- oder Erdalkalilauge in Ameisensäure und Methanol überführt.

Die vorliegende Erfindung beschreibt ein Verfahren zur Aufarbeitung der Gemische/Mischungen durch Umsetzung mit konzentrierten insbesondere wässrigen Alkali- oder Erdalkalihydroxiden, bei denen der Formaldehydanteil durch Disproportionierung in Ameisensäure und Methanol überführt wird, die gesamte Ameisensäure zu den entsprechenden Formiaten neutralisiert und das gebildete Methanol destillativ leicht abgetrennt werden kann. Gleichzeitig können unerwünschte Verunreinigungen, wie beispielsweise organische Aminverbindungen, in verwertbare Stoffe überführt und abgetrennt werden. Die erhaltenen Alkali- oder Erdalkaliformiate wiederum sind gefragte Produkte in vielfältigen Anwendungen, das gebildete Methanol kann zusammen mit dem bereits in der Lösung enthaltenen Methanol wieder in den Stoffkreislauf zurückgeführt werden. Der Wasseranteil enthält nur noch einen sehr geringen Anteil an organischen Verbindungen und ist daher in biologischen Abwasseranlagen leicht zu entsorgen.

Das Verfahren basiert auf der Umsetzung von Formaldehyd mit Alkali- oder Erdalkalihydroxiden zu Ameisensäure und Methanol. 1 Mol Formaldehyd disproportioniert hier zu 0,5 Mol Ameisensäure und 0,5 Mol Methanol. Die Disproportionierung wird durch die entsprechenden Alkali- oder Erdalkalihydroxide katalysiert, die entstehende Ameisensäure reagiert sofort unter Wasserbildung zu den entsprechenden Formiaten, während das entstehende Methanol zusammen mit dem bereits in der Lösung enthaltenen Methanol leicht destillativ abgetrennt werden kann. Die in den Lösungen enthaltene Ameisensäure reagiert sofort mit den Alkali- oder Erdalkalihydroxiden zu den korrespondierenden Formiaten. Dies setzt voraus, dass man die entsprechenden Alkali- oder Erdalkalihydroxide vorlegt.

Als Alkali- oder Erdalkalihydroxide können insbesondere Natriumhydroxid, Kaliumhydroxid oder Calciumhydroxid verwendet werden. Daraus entstehen dann Natriumformiat, Kaliumformiat oder Calciumdiformiat. Diese Produkte finden beispielsweise als Futtermitteladditive, Silierhilfsmittel, Erstarrungs- oder Abbindebeschleuniger bei zementgebundenen Materialien wie Fliesenkleber, Enteisungsmitteln für Flugfelder, Konservierungsmittel für Lebens- oder Futtermittel oder als Additiv in der Erdölförderung vielfältigen Einsatz. Das entstehende Methanol kann destillativ ohne großen Aufwand in hoher Reinheit abgetrennt und als Rohstoff für die Formaldehydherstellung, als Kohlenstoffträger für Kläranlagen oder als Lösungsmittel wiederverwendet werden. Die entstehenden Abwässer sind entsprechend gering organisch belastet und enthalten praktisch kein toxisches Formaldehyd mehr und lassen sich somit einfach über biologische Abwasserreinigungsanlagen entsorgen.

Gemäß einer bevorzugten Ausführungsform der vorliegenden Erfindung wird ein Inhibitor gegen die parallel verlaufende Aldolkondensation von Formaldehyd eingesetzt, insbesondere mit dem Alkali- und/oder Erdalkalihydroxid vorgelegt. Besonders bevorzugt im Sinne der vorliegenden Erfindung wird hierbei Natriumtetraborat, insbesondere das Dekahydrat (Na₂B₄O₇ x 10 H₂O) eingesetzt. Damit wird insbesondere eine während der Reaktion möglicherweise auftretende Gelb- bzw. Braunfärbung vermieden.

Zur Disproportionierung von Formaldehyd in Ameisensäure und Methanol ist prinzipiell wenigstens eine äquimolare Menge an Alkali- und/oder Erdalkalihydroxid erforderlich. Da es sich hierbei jedoch um eine Gleichgewichtsreaktion handelt, wird zur Verschiebung des Gleichgewichts in Richtung der Produkte vorzugsweise ein Überschuss, insbesondere ein hoher Stoffmengenüberschuss an Alkali- und/oder Erdalkalihydroxid eingesetzt. Besonders bevorzugt im Sinne der vorliegenden Erfindung wird ein Stoffmengenüberschuss an Alkali- und/oder Erdalkalihydroxid von wenigstens 10 Mol-% eingesetzt, insbesondere vorgelegt.

Besonders bevorzugt wird die erfindungsgemäße Reaktion unter erhöhtem Druck durchgeführt. Diese baut sich aufgrund der Exothermie der Reaktion selbstständig auf, kann jedoch auch bewusst erzeugt werden.

### Beispiele;

Anhand der nachfolgenden Beispiele wird das erfindungsgemäße Verfahren weiter erläutert:

### Beispiel 1:

### Aufarbeitung einer wässrigen Ameisensäurelösung mit 70,2 Gew.% Ameisensäure, 5,3 Gew.% Formaldehyd und 5,3 Gew.% Methanol zu Kaliumformiatlösung

In einem 1 l Rührgefäß mit Dosiervorlage, Heiz- und Kühlmantel und 10 cm Destillationskolonne wurden 5,0 Mol Kaliumhydroxidlauge in Form von 560 g 50 Gew.%iger wässriger Lösung und 1,90 g (0,005 Mol) Natriumtetraborat (Na₂B₄O₇ x 10 H₂O) vorgelegt. In diese Lösung wurde unter Rühren in ca. 60 Minuten 282 g des aufzuarbeitenden Ameisensäuregemischs, enthaltend 4,3 Mol Ameisensäure (entsprechend 198 g), 0,5 Mol Formaldehyd (entsprechend 15 g) und 0,5 Mol Methanol (entsprechend 15 g) zudosiert. Bei der Berechnung der Zugabemenge werde darauf geachtet, dass die Summe der Stoffmenge in Mol aus Ameisensäure + (Formaldehyd ÷2) jeweils gleich der Stoffmenge in Mol Kalilauge x 0,9 betrug.

Die Reaktionslösung erwärmte sich dabei sehr stark und begann zu sieden. Nach beendeter Zugabe der Säuremischung wurde für 60 Minuten bei dieser Temperatur und unter leichtem Rückfluss gehalten. Mit ca. 24 g (0,5 Mol) Ameisensäure in Form von 94 %iger Lösung wurde der pH Wert auf ca. 9 bis 10 eingestellt und anschließend ein Destillat aus ca. 70 Gew.% (0,75 Mol) Methanol und 30 Gew.% Wasser abgetrennt. Aus der verbleibenden Lösung wurde so viel Wasser abgetrennt, dass ca. 600 g einer 70 Gew.%igen Kaliumformiatlösung im Rührgefäß verblieben.

### Beispiel 2:

### Aufarbeitung einer wässrigen Ameisensäure / Formaldehyd / Methanol Mischung mit 0,4 Gew.% Ameisensäure, 24 Gew.% Formaldehyd und 2 Gew.% Methanol zu Kaliumformiatlösung

In einem 2 l Rührgefäß mit Dosiervorlage, Heiz- und Kühlmantel und 10 cm Destillationskolonne wurden 5,0 Mol Kaliumhydroxidlauge in Form von 560 g 50 Gew.%iger wässriger Lösung und 1,90 g (0,005 Mol) Natriumtetraborat (Na₂B₄O₇ x 10 H₂O) vorgelegt. In diese Lösung wurde unter Rühren in ca. 60 Minuten 1.100 g des aufzuarbeitenden Ameisensäuregemischs, enthaltend 0,1 Mol Ameisensäure (entsprechend 4,4 g), 8,8 Mol Formaldehyd (entsprechend 264 g) und 0,7 Mol Methanol (entsprechend 22 g) zudosiert. Bei der Berechnung der Zugabemenge wurde darauf geachtet, dass die Summe der Stoffmenge in Mol aus Ameisensäure + (Formaldehyd ÷2) jeweils gleich der Stoffmenge in Mol Lauge x 0,9 betrug.

Die Reaktionslösung erwärmte sich dabei und begann zu sieden. Nach beendeter Zugabe der Säuremischung wurde für 60 Minuten bei dieser Temperatur und unter leichtem Rückfluss gehalten. Mit ca. 24 g (0,5 Mol) Ameisensäure in Form von 94 Gew.%iger Lösung wurde der pH Wert auf ca. 9 bis 10 eingestellt und anschließend ein Destillat aus ca. 70 Gew.% (0,8 Mol) Methanol und 30 Gew.% Wasser abgetrennt. Aus der verbleibenden Lösung wurde so viel Wasser abgetrennt, dass ca. 840 g einer 70 Gew.-%igen Kaliumformiatlösung im Rührgefäß verblieben.

### Beispiel 3:

### Aufarbeitung einer wässrigen Ameisensäurelösung mit 66,6 Gew.% Ameisensäure, 6,2 Gew.% Formaldehyd und 5,2 Gew.% Methanol zu Natriumformiatlösung

In einem 1 l Rührgefäß mit Dosiervorlage, Heiz- und Kühlmantel und 10 cm Destillationskolonne wurden 5,0 Mol Natriumhydroxidiauge in Form von 400 g 50 Gew.%iger wässriger Lösung und 1,90 g (0,005 Mol) Natriumtetraborat (Na₂B₄O₇ x 10 H₂O) vorgelegt. In diese Lösung wurde unter Rühren in ca. 60 Minuten 290 g des aufzuarbeitenden Ameisensäuregemischs, enthaltend 4,2 Mol Ameisensäure (entsprechend 193 g), 0,6 Mol Formaldehyd (entsprechend 18 g) und 0,5 Mol Methanol (entsprechend 15 g) zu dosiert. Bei der Berechnung der Zugabemenge wurde darauf geachtet, dass die Summe der Stoffmenge in Mol aus Ameisensäure + (Formaldehyd ÷2) jeweils gleich der Stoffmenge in Mol Kalilauge x 0,9 betrug.

Die Reaktionslösung erwärmte sich dabei sehr stark und begann zu sieden. Nach beendeter Zugabe der Säuremischung wurde für 60 Minuten bei dieser Temperatur und unter leichtem Rückfluss gehalten. Mit ca. 24 g (0,5 Mol) Ameisensäure in Form von 94 Gew.%iger Lösung wurde der pH Wert auf ca. 9 bis 10 eingestellt und anschließend wird ein Destillat aus ca. 70 Gew.% (0,8 Mol) Methanol und 30 Gew.% Wasser abgetrennt. Aus der verbleibenden Lösung wurde so viel Wasser abgetrennt, dass ca. 680 g einer 50 Gew.%igen Natriumformiatlösung im Rührgefäß verbleiben.

### Beispiel 4:

### Aufarbeitung einer wässrigen Ameisensäurelösung mit 66,6 Gew.% Ameisensäure, 6,2 Gew.% Formaldehyd und 5,2 Gew.% Methanol zu Calciumdiformiat

In einem 1 l Rührgefäß mit Dosiervorlage, Heiz- und Kühlmantel und 10 cm Destillationskolonne wurden 2,5 Mol Calciumhydroxid in Form von 462,5 g einer 40 Gew.%igen wässrigen Suspension und 1,90 g (0,005 Mol) Natriumtetraborat (Na₂B₄O₇ x 10 H₂O) vorgelegt. In diese Suspension wurde unter Rühren in ca. 60 Minuten 290 g des aufzuarbeitenden Ameisensäuregemischs, enthaltend 4,2 Mol Ameisensäure (entsprechend 193 g), 0,6 Mol Formaldehyd (entsprechend 18 g) und 0,5 Mol Methanol (entsprechend 15 g) zu dosiert. Bei der Berechnung der Zugabemenge wurde darauf geachtet, dass die Summe der Stoffmenge in Mol aus Ameisensäure + (Formaldehyd ÷2) jeweils gleich der Stoffmenge in Mol Calciumhydroxid x 1,8 ist.

Die Reaktionslösung erwärmte sich dabei sehr stark und begann zu sieden. Nach beendeter Zugabe der Säuremischung wurde für 180 Minuten bei dieser Temperatur und unter leichtem Rückfluss gehalten. Mit ca. 24 g (0,5 Mol) Ameisensäure in Form von 94 Gew.%iger Lösung wurde der pH Wert auf ca. 9 - 10 eingestellt und anschließend ein Destillat aus ca. 70 % (0,8 Mol) Methanol und 30 % Wasser abgetrennt. Die verbleibende Suspension wurde völlig eingedampft und getrocknet. Es resultierten ca. 325 g trockenes Calciumdiformiat in Form von weißen Kristallen.

### Beispiel 5:

### Aufarbeitung einer wässrigen Ameisensäure / Formaldehyd / Methanol Mischung mit 0,4 % Ameisensäure, 24 % Formaldehyd und 2 % Methanol zu Calciumdiformiat

In einem 1 l Rührgefäß mit Dosiervorlage, Heiz- und Kühlmantel und 10 cm Destillationskolonne wurden 2,5 Mol Calciumhydroxid in Form von 462,5 g einer 40 Gew.-%igen wässrigen Suspension und 1,90 g (0,005 Mol) Natriumtetraborat (Na₂B₄O₇ x 10 H₂O) vorgelegt. In diese Suspension wurde unter Rühren in ca. 60 Minuten 1.100 g des aufzuarbeitenden Ameisensäuregemischs, enthaltend 0,1 Mol Ameisensäure (entsprechend 4,4 g), 8,8 Mol Formaldehyd (entsprechend 264 g) und 0,7 Mol Methanol (entsprechend 22 g) zu dosiert. Bei der Berechnung der Zugabemenge wurde darauf geachtet, dass die Summe der Stoffmenge in Mol aus Ameisensäure + (Formaldehyd ÷2) jeweils gleich der Stoffmenge in Mol Calciumhydroxid x 1,8 ist.

Die Reaktionslösung erwärmte sich dabei sehr stark und begann zu sieden. Nach beendeter Zugabe der Säuremischung wurde für 180 Minuten bei dieser Temperatur und unter leichtem Rückfluss gehalten. Mit ca. 24 g (0,5 Mol) Ameisensäure in Form von 94 Gew.%iger Lösung wurde der pH Wert auf ca. 9 bis 10 eingestellt und anschließend wird ein Destillat aus ca. 70 Gew.% (0,8 Mol) Methanol und 30 Gew.% Wasser abgetrennt. Die verbleibende Suspension wurde völlig eingedampft und getrocknet.

Es resultierten ca. 325 g trockenes Calciumdiformiat in Form von weißen Kristallen.

### Beispiel 6:

### Aufarbeitung einer wässrigen Ameisensäurelösung mit 70,2 Gew.% Ameisensäure, 5,3 Gew.% Formaldehyd und 5,3 Gew.% Methanol zu Kaliumformiatlösung

In einem 1 l Edelstahl - Druckgefäß mit Dosiervorlage, Heiz- und Kühlmantel und absperrbarem 20 cm Brüdenkühler wurden 5,0 Mol Kaliumhydroxidlauge in Form von 560 g 50 Gew.%iger wässriger Lösung und 1,90 g (0,005 Mol) Natriumtetraborat (Na₂B₄O₇ x 10 H₂O) vorgelegt. In diese Lösung wurde unter Rühren in ca. 60 Minuten 282 g des aufzuarbeitenden Ameisensäuregemischs, enthaltend 4,3 Mol Ameisensäure (entsprechend 198 g), 0,5 Mol Formaldehyd (entsprechend 15 g) und 0,5 Mol Methanol (entsprechend 15 g) zudosiert. Bei der Berechnung der Zugabemenge wurde darauf geachtet, dass die Summe der Stoffmenge in Mol aus Ameisensäure + (Formaldehyd ÷2) jeweils gleich der Stoffmenge in Mol Kalilauge x 0,9 betrug.

Die Reaktionslösung erwärmte sich dabei sehr stark bis auf 120 °C. Es baute sich ein Druck von ca. 4 bar auf. Nach beendeter Zugabe der Säuremischung wurde für 60 Minuten bei dieser Temperatur gehalten. Mit ca. 24 g (0,5 Mol) Ameisensäure in Form von 94 %iger Lösung wurde der pH Wert auf ca. 9 bis 10 eingestellt und anschließend wurde der bestehende Druck in kontrolliert in den Brüdenkühler entspannt. Dabei wurde zunächst ein Destillat aus ca. 70 Gew.% (0,75 Mol) Methanol und 30 Gew.% Wasser abgetrennt, um es ggfs. wieder in den Stoffkreislauf zu überführen. Die Reaktionslösung kühlte sich dabei auf ca. 100 °C ab. Aus der verbleibenden Lösung wurde destillativ so viel Wasser abgetrennt, dass ca. 600 g einer 70 Gew.%igen Kaliumformiatlösung im Rührgefäß verblieben.

## Patentansprüche

1. Verfahren zur Aufarbeitung von Gemischen, die Ameisensäure, Formaldehyd und Methanol enthalten, **dadurch gekennzeichnet, dass** man den Ameisensäureanteil mit Lauge direkt in die entsprechenden Formiate und zeitgleich den Formaldehydanteil nach Disproportionierung in Gegenwart von Alkali- oder Erdalkalilauge in Ameisensäure und Methanol überführt.

2. Verfahren nach Anspruch 1, **dadurch gekennzeichnet, dass** man als Lauge wässrige Lösungen von Natriumhydroxid, Kaliumhydroxid oder Suspensionen von Calciumhydroxid einsetzt.

3. Verfahren nach Anspruch 1 oder 2, **dadurch gekennzeichnet, dass** man Natriumtetraborat als Inhibitor einsetzt.

4. Verfahren nach einem der Ansprüche 1 bis 3, **dadurch gekennzeichnet, dass** man die Reaktion jeweils bei einem Stoffmengenüberschuss an Alkali- und/oder Erdalkalihydroxid, insbesondere einen Stoffmengenüberschuss von wenigstens 10 Mol - % durchführt.

5. Verfahren nach Anspruch 1, **dadurch gekennzeichnet, dass** man die Reaktion in druckfesten Reaktoren durchführt wird und insbesondere den sich durch die Reaktionsentalphie ergebende Temperatur- und Druckanstieg nach Abschluss der Reaktion durch kontrolliertes Entspannen zur Destillation von Methanol/Wasser Gemischen nutzt.

## Claims

1. A process for reprocessing mixtures containing formic acid, formaldehyde and methanol, **characterized in that** the formic acid fraction is directly transformed into the corresponding formates with a base, and at the same time, the formaldehyde fraction is transformed into formic acid and methanol after disproportionation in the presence of aqueous alkali metal hydroxide or alkaline earth metal hydroxide.

2. The process according to claim 1, **characterized in that** aqueous solutions of sodium hydroxide, potassium hydroxide or suspensions of calcium hydroxide are employed as said base.

3. The process according to claim 1 or 2, **characterized in that** sodium tetraborate is employed as an inhibitor.

4. The process according to any of claims 1 to 3, **characterized in that** the reaction is respectively performed at a molar excess of alkali metal and/or alkaline earth metal hydroxide, especially at a molar excess of at least 10 mole percent.

5. The process according to claim 1, **characterized in that** the reaction is performed in pressure-resistant reactors, and in particular, the increase of temperature and pressure resulting from the reaction enthalpy is utilized for the distillation of methanol/water mixtures by controlled pressure-release after completion of the reaction.

## Revendications

1. Procédé pour le retraitement de mélanges contenant de l'acide formique, du formaldéhyde et du méthanol, **caractérisé en ce que** la fraction d'acide formique est transformée directement en les formiates correspondants avec une base, et en même temps, la fraction de formaldéhyde est transformée, après dismutation, en acide formique et méthanol en présence d'un hydroxyde de métal alcalin ou alcalino-terreux aqueux.

2. Procédé selon la revendication 1, **caractérisé en ce que** des solutions aqueuses d'hydroxyde de sodium, d'hydroxyde de potassium ou des suspensions d'hydroxyde de calcium sont utilisés en tant que base.

3. Procédé selon la revendication 1 ou 2, **caractérisé en ce que** du tétraborate de sodium est utilisé en tant qu'inhibiteur.

4. Procédé selon l'une quelconque des revendications 1 à 3, **caractérisé en ce que** la réaction est effectuée respectivement avec un excès molaire d'hydroxyde de métal alcalin et/ou alcalino-terreux, notamment avec un excès molaire d'au moins 10 pour cent molaire.

5. Procédé selon la revendication 1, **caractérisé en ce que** la réaction est effectuée dans des réacteurs résistants à la pression, et notamment, l'augmentation de la température et de la pression résultant de l'enthalpie de réaction est utilisée pour la distillation de mélanges méthanol-eau par libération de pression commandée après la fin de la réaction.
